(19) Europäisches Patentamt
European Patent Office
Office européen des brevets

(11) EP 1 153 006 B1

(12) # EUROPÄISCHE PATENTSCHRIFT

(45) Veröffentlichungstag und Bekanntmachung des Hinweises auf die Patenterteilung:
**05.11.2003 Patentblatt 2003/45**

(21) Anmeldenummer: **00912443.9**

(22) Anmeldetag: **02.02.2000**

(51) Int Cl.[7]: **C07C 41/46**

(86) Internationale Anmeldenummer:
**PCT/EP00/00821**

(87) Internationale Veröffentlichungsnummer:
**WO 00/048974 (24.08.2000 Gazette 2000/34)**

(54) **STABILISIERTE ZUSAMMENSETZUNGEN VON O- UND N-VINYLVERBINDUNGEN UND VERWENDUNG VON AMMONIUMSALZEN ALS STABILISIERUNGSMITTEL**

STABILISED COMPOSITIONS OF O- AND N-VINYL COMPOUNDS AND THE USE OF AMMONIUM SALTS AS STABILISERS

COMPOSITIONS STABILISEES DE COMPOSES O- ET N-VINYLE ET UTILISATION DE SELS D'AMMONIUM COMME AGENTS DE STABILISATION

(84) Benannte Vertragsstaaten:
**AT BE CH CY DE DK ES FI FR GB GR IE IT LI LU MC NL PT SE**

(30) Priorität: **16.02.1999 DE 19906316**

(43) Veröffentlichungstag der Anmeldung:
**14.11.2001 Patentblatt 2001/46**

(73) Patentinhaber: **BASF AKTIENGESELLSCHAFT**
**67056 Ludwigshafen (DE)**

(72) Erfinder:
- **PREISS, Thomas**
  **D-67065 Ludwigshafen (DE)**
- **LORENZ, Rudolf, Erich**
  **D-67069 Ludwigshafen (DE)**
- **WEIGUNY, Sabine**
  **D-67251 Freinsheim (DE)**
- **HENKELMANN, Jochem**
  **D-68165 Mannheim (DE)**

(74) Vertreter: **Isenbruck, Günter, Dr.**
**Isenbruck, Bösl, Hörschler, Wichmann, Huhn, Patentanwälte**
**Theodor-Heuss-Anlage 12**
**68165 Mannheim (DE)**

(56) Entgegenhaltungen:
**DE-A- 19 609 312**
**DE-B- 1 286 015**
**US-A- 5 691 462**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die Erfindung betrifft stabilisierte Zusammensetzungen von O- und N-Vinylverbindungen, ein Verfahren zur Stabilisierung von O- und N- Vinylverbindungen und die Verwendung der Stabilisierungsmittel.

Vinylverbindungen, bei denen die Vinyl-Gruppe oder die Vinyl-Gruppen über Sauerstoff oder Stickstoff mit dem Grundgerüst der Verbindung verbunden sind, sogenannte O- und N-Vinylverbindungen, neigen zur spontanen stark exothermen kationischen Polymerisation, insbesondere bei Anwesenheit von Säure. Darüber hinaus ist bei Vinylethern eine schleichend fortschreitende Polymerisation zu Polyvinylethern bzw. bei Vinylaminen/amiden zu Polyvinylaminen/amiden zu beobachten. Auch eine Lagerung unter inerten Bedingungen verhindert die Polymerisation nicht.

O-Vinylether, die wirtschaftlich von besonderer Bedeutung sind, werden als Monomere bei der Herstellung zahlreicher homo- oder copolymerisierter Produkte eingesetzt oder dienen als Reaktivverdünner in Oberflächenbeschichtungen. Bei diesem Anwendungsgebiet ist die Verhinderung der vorzeitigen, Polymerisation essentiell. Bei der Lagerung von O-Vinylethern unter inerten, Bedingungen lassen sich bereits nach wenigen Wochen mit Hilfe der Gel-Permeations-Chromatographie (GPC) Polyvinyletheroligomere nachweisen. Der durch Gaschromatographie (GC) ermittelbare Gehalt an Monomeren nimmt entsprechend ab. Ebenso ist die Bildung von giftigen Acetaldehyd ein weiterer Hinweis auf die Zersetzung des O-Vinylethers. Ohne Zusatz eines Stabilisators beträgt der Acetaldehydgehalt zum Teil schon nach wenigen Wochen 800 ppm und mehr. Enthalten O-Vinylether noch weitere funktionelle Gruppen wie Hydroxy-Gruppen, so ist eine spontane intra-, wie auch intermolekulare Acetalisierung möglich.

Als Stabilisator sind seit langern Alkalimetallhydroxide, speziell KOH bekannt (Ullmann's Encyclopedia of Industrial Chemistry, Band 27, 5. Auflage, Seilen 435 bis 441, speziell Seite 440, VCH-Verlagsgesellschaft mbH, Weinheim). Allerdings sind diese, zum Beispiel KOH, nicht in allen Vinylethern löslich, was dazu führt, daß relativ große Mengen an Hydroxiden zugesetzt werden müssen, die sich am Boden der Gefäße abscheiden. Der Einsatz von größeren Mengen an Hydroxiden ist problematisch, da die Hydroxide beträchtliche Mengen Wasser enthalten und durch zusätzlich durch Deprotonierung von Alkoholen Wasser zum Teil freisetzen. Dieses Wasser bewirkt den Zerfall von Vinylethern in Acetaldehyd und Alkohol. Acetaldehyd ist tendenziell giftig und führt zu einer geruchlichen Beeinträchtigung des Produkts. Weiter ist bekannt, daß der Einsatz von KOH eine farbliche Beeinträchtigung des Produktes bewirkt. Dies führt zu einer Beeinträchtigung der Anwendungsbreite und zur Verringerung der maximalen Lagerzeit von Vinylethern.

Aus US-A 5,691,462 ist bekannt, daß die Alkalimetallsalze einer unsubstiuierten $C_1$-$C_{14}$-Carbonsäure als Stabilisatoren für Vinylether eingesetzt werden können. Zum Beispiel werden Natrium- und Kaliumacetat und Natrium- und Kaliumcarbonat genannt. Zwar weisen die mit diesen Substanzen stabilisierten Vinyletherzusammensetzungen eine im Vergleich zu KOH-stabilisierten Zusammensetzungen höhere Farbstabilität auf, jedoch läßt die Unterdrückung der Acetaldehydbildung zu wünschen übrig.

In DE-A 1 286 015 werden aromatische Vinylverbindungen wie Styrol gegen Polymerisation dadurch stabilisiert, daß Hydroxylammoniumacetate der allgemeinen Formel $CH_3COO[NH(OH)R_1R_2]$, in der $R_1$ und $R_2$ je ein Alkylrest mit 1 bis 6 Kohlenstoffatomen bedeuten, als Stabilisierungsmittel zugesetzt werden.

In DE-A 196 09 312 werden Monomerenzusammensetzungen, die Monomere mit bestimmten Vinylgruppen aufweisen, durch Zusatz von N-Oxyl-Verbindungen eines sekundären Amins, welches keine Wasserstoffatome an den $\alpha$-C-Atomen trägt, gegen Polymerisation stabilisiert.

Es ist Aufgabe der Erfindung, stabilisierte Zusammensetzungen von Vinylverbindungen anzugeben, die einerseits eine gute Farbstabilität aufweisen und andererseits eine geringe Zersetzungsgeschwindigkeit zeigen. Eine weitere Aufgabe der Erfindung ist die Angabe von Stoffen, welche als stabilisierende Zusätze zu Vinylverbindungen verwandt werden können und die den daraus resultierenden Zusammensetzungen die genannten Eigenschaften verleihen. Die Zusätze sollten preiswert sein, eine befriedigende Löslichkeit aufweisen und zudem von den Vinylverbindungen abtrennbar sein.

Die Erfindung ist dann eine Zusammensetzung von Vinylverbindungen enthaltend

a) mindestens eine polymerisierbare Verbindung, die mindestens eine O-Vinylgruppe oder eine N-Vinylgruppe aufweist, und
b) mindestens ein Ammoniumsalz der allgemeinen Formel (I)

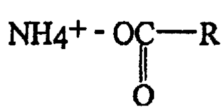

(I),

wobei R $NH_2$, $O^-$ $NH_4^+$, $C_1$-$C_{18}$-Alkyl, $C_6$-$C_{18}$-Cycloalkyl, oder $C_7$-$C_{18}$-Alkylcycloalkyl bedeutet.

Ammoniumsalze der allgemeinen Formel (I) lassen sich relativ wasserfrei herstellen und sind preiswert. Es wurde gefunden, daß der Zusatz dieser Ammoniumsalze zu den polymerisierbaren Verbindungen der Komponente a) die vorzeitige Polymerisation dieser Verbindungen unterdrückt, wobei die Farbveränderung der resultierenden erfindungsgemäßen Zusammensetzung deutlich geringer ist als bei Zusatz der bekannten Verbindungen. Darüber hinaus wurde festgestellt, daß sich die erfindungsgemäßen Ammoniumsalze der allgemeinen Formel (I) relativ gut in den polymerisierbaren Verbindungen der Komponente a) lösen bzw. dispergieren lassen und trotzdem gut abtrennbar sind. Die resultierenden erfindungsgemäßen Zusammensetzungen sind gut pumpbar.

Unter einer polymerisierbaren Verbindung, die mindestens eine O-Vinylgruppe oder eine N-Vinylgruppe aufweist, wird ein heterosubstituiertes Vinylmonomeres verstanden, das als Heteroatom an der Vinyl-Gruppe Sauerstoff oder Stickstoff trägt. Als heterosubstituierte Vinylmonomere kommen beispielsweise Vinylcarbonsäureester wie Vinylacetat, Vinylpropionat oder Vinylbutyrat, Vinylether wie Methylvinylether, Ethylvinylether oder Butylvinylether, Triethylenglykoldivinylether, Hydroxyalkylvinylether wie Hydroxybutylvinylether und Cycloalkylvinylether wie Cyclohexylvinylether, ferner Vinylcarbazole, Vinylpyrrolidone, Vinylphthalimide, Vinylcaprolactame, Vinylimidazole und Vinylformamid in Betracht.

Bevorzugte Ammoniumsalze sind Ammoniumcarbamat, Ammoniumcarbonat und die Ammoniumsalze der Methan-, Ethan-, Propan-, Isopropan-, Butan-, iso-Butan-, tert-Butan-, Pentan-, iso-Pentan-, Hexan- oder Cyclohexansäuren und Gemische davon.
Besonders bevorzugte Ammoniumsalze sind Ammoniumcarbonat ($(NH_4)_2CO_3$) oder Ammoniumcarbamat ($NH_4NH_2CO_2$).

Bei einer weiteren Ausführungsform der erfindungsgemäßen Zusammensetzung fällt mindestens eine polymerisierbare Verbindung der Komponente a) unter die allgemeine Formel (II)

$$CH_2{=}CH—X—R^1 \qquad (II),$$

wobei die Symbole die folgende Bedeutung haben

X O oder $NR^2$,

$R^1$

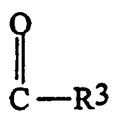

oder $R^3$,

$R^2$ Wasserstoff oder $C_1$-$C_4$-Alkyl oder gemeinsam mit $R^3$ eine $C_3$-, $C_4$- oder $C_5$-Alkylenbrücke oder Alkenylenbrücke bildend, in welchen bis zu zwei nicht-benachbarte $CH_2$-Gruppen durch NH, N($C_1$-$C_4$-Alkyl), N($C_6$-$C_{10}$-Aryl) oder Sauerstoff und bis zu zwei nicht-benachbarte CH-Gruppen durch N ersetzt sein können und

$R^3$ Wasserstoff oder eine einfach oder mehrfach Hydroxy-substituierte oder Vinyloxy-substituierte oder unsubstituierte $C_1$-$C_{16}$-Alkyl-, $C_6$-$C_{16}$-Cycloalkyl- oder $C_1$-$C_4$-Alkyl-$C_6$-$C_{12}$-cycloalkylgruppe oder zusammen mit $R^2$ eine $C_3$-, $C_4$- oder $C_5$-Alkylenbrücke oder Alkenylenbrücke darstellend, in welchen bis zu zwei nicht-benachbarte $CH_2$-Gruppen durch NH, N($C_1$-$C_4$-Alkyl), N($C_6$-$C_{10}$-Aryl) oder Sauerstoff und bis zu zwei nicht-benachbarte CH-Gruppen durch N ersetzt sein können.

Bei den polymerisierbaren Verbindungen der allgemeinen Formel (II), die in der erfindungsgemäßen Zusammensetzung enthalten sind, bedeutet X bevorzugt Sauerstoff. Vinylether, in denen $R^1$ eine $C_1$-$C_4$-Alkylgruppe, also Methyl, Ethyl, n-Propyl, iso-Propyl, n-Butyl, iso-Butyl, sec-Butyl oder tert-Butyl ist, stellen bevorzugte polymerisierbare Verbindungen dar. Ferner sind Vinylether bevorzugt, bei denen $R^1$ eine $C_1$-$C_4$-Hydroxyalkylgruppe oder eine $C_1$-$C_4$-Alkylgruppe, die einfach Vinyloxy-substituiert ist, darstellt.

Weitere Ausführungsformen der erfindungsgemäßen Zusammensetzung sind solche, die als polymerisierbare Verbindung Triethylenglykoldivinylether (DVE), 4-Hydroxybutylvinylether und/oder Cyclohexylvinylether enthalten. Besitzt X die Bedeutung $NR^2$, so ist $R^1$ vorzugsweise eine CO-$R^3$-Gruppe.

Als Reste $R^3$ kommen neben Wasserstoff und den genannten $C_1$-$C_4$-Alkylgruppen auch solche Reste in Betracht, die zusammen mit $NR^2$ einen gesättigten oder ungesättigten 5- bis 7- gliedrigen Ring bilden. Beispiele solcher

Ringsysteme sind solche der allgemeinen Formel (III)

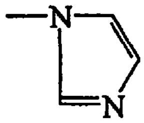 , 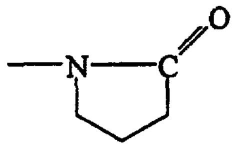 oder 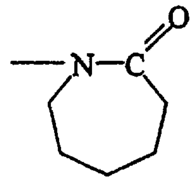 (III).

Bei einer weiteren Ausführungsform der erfindungsgemäßen Zusammensetzung stellt X-$R_1$ $NH_2COCH_3$, N-Imidazolyl, N-Pyrrolidinonyl oder N-Caprolactamyl dar.

Ein weiterer Gegenstand der Erfindung ist die Verwendung eines Ammoniumsalzes der allgemeinen Formel (I) als Zusatz zu polymerisierbaren Verbindungen, die mindestens eine O-Vinylgruppe oder eine N-Vinylgruppe aufweisen.

Eine weitere Ausführungsform der Erfindung ist die Verwendung von Ammoniumcarbonat ($(NH_4)_2CO_3$) oder von Ammoniumcarbamat ($NH_4NH_2CO_2$) als Zusatz zu polymerisierbaren Verbindungen, die mindestens eine O-Vinylgruppe oder eine N-vinylgruppe aufweisen.

Ein weiterer Gegenstand der Erfindung ist ein Verfahren zur Verhinderung der vorzeitigen Polymerisation von Zusammensetzungen, die polymerisierbare Verbindungen enthalten, die mindestens eine O-Vinylgruppe oder eine N-Vinylgruppe aufweisen, das den Zusatz eines Ammoniumsalzes der allgemeinen Formel (I) zu den Zusammensetzungen umfaßt.

Bei einer weiteren Ausführungsform des erfindungsgemäßen Verfahrens wird als Ammoniumsalz Ammoniumcarbonat ($(NH_4)_2CO_3$) oder Ammoniumcarbamat ($NH_4NH_2CO_2$) eingesetzt.

Die Erfindung wird durch die Beispiele näher beschrieben.

**Beispiel 1**

Frisch destillierter Triethylenglykoldivinylether (DVE-3) eines Gehalts von 98,8 Flächen-% bzw. 99,8 Flächen-% (GC) und einer Farbzahl <5 APHA, der in der Gel-Permeations-Chromatographie (GPC) keine oligomeren Verunreinigungen enthält, wird über einen längeren Zeitraum unter Schutzgas, also unter inerten Bedingungen, in einer braunen Gasflasche bei Raumtemperatur (23°C) bzw. bei 50°C gelagert. Die Ergebnisse ergeben sich aus Tabelle 1.1 und 1.2:

Tabelle 1.1

| DVE-3 unstabilisiert 50°C | | | |
|---|---|---|---|
| | DVE-3-FL% | APHA | AA |
| unmittelb. n. Zugabe | 98,8 | 4 | <5 |
| 2. Woche | 98,5 | 2 | 44 |
| 4. Woche | 98,6 | 0 | 110 |
| 6. Woche | 98,7 | 9 | 160 |
| 8. Woche | 98,5 | 6 | 160 |
| 10. Woche | 98,9 | 5 | 200 |
| Polymere Bestandteile (GPC): 11554 (nach 8 Wochen) | | | |

Tabelle 1.2

| DVE-3 unstabilisiert bei Raumtemperatur | | | |
|---|---|---|---|
| | DVE-3 FL% | APHA | AA |
| unmittelb.n. Zugabe | 99,81 | <5 | <5 |
| 4. Woche | 99,76 | 7 | 6 |
| 6. Woche | 99,77 | 14 | 12 |
| 12. Woche | 99,75 | 7 | 25 |

Tabelle 1.2 (fortgesetzt)

| DVE-3 unstabilisiert bei Raumtemperatur | | | |
|---|---|---|---|
| | DVE-3 FL% | APHA | AA |
| 16. Woche | 99,73 | 7 | 31 |
| 20. Woche | 99,74 | 6 | 40 |
| 24. Woche | 99,74 | 5 | n.b. |
| 28. Woche | 99,65 | 11 | 45 |

Polymere Bestandteile (GPC): 50 (nach 16 Wochen, Raumtemperatur)

n.b. nicht bestimmt

AA: AA bezeichnet den Acetaldehydgehalt in ppm. Die Bestimmung erfolgte durch eine Headspace-Kapillar-GC-Apparatur mit Flammenionisationsdetektion (FID). Die Trennung wurde auf einer mit 100%-Polydimethylsiloxan belegten Fused-Silica-Kapillare durchgeführt. Die Quantifizierung erfolgte nach der Standardadditionsmethode.
GC-Säule: Fused-Silica-Kapillare belegt mit 100% Polydimethylsiloxan als stationäre Phase z.B. DB 1 der Fa. J&W Scientific, Länge 30 m, Innnedurchmesser 0,25mm, Filmdicke 1µm
Probeneinwage: Die Probeneinwage wurde an die zu bestimmenden Massenanteile angepaßt.
Kalibrierung: Die Kalibrierung erfolgte durch Zugabe definierter Mengen geeignet konzentrierter Standardlösungen von Acetaldehyd gelöst in Dimethylacetamid zu separat eingewogenen Probenportionen.
Analyseparameter: Temperierungsbedingungen für die Headspace-GC-Apparatur: 40°C, Transferleitungstemperatur: 120°C, Probenaufgabe: 6 Sekunden Injektionszeit, Ofenheizprogramm: 5 min. isotherm bei 50°C, 50°C-230°C mit 5K/min, 10 min. isotherm bei 230°C, Temperatur des Detektors (FID): 250°C, Trägergas: Helium (Vordruck 0,7 bar), Split etwa 10 ml/min

APHA: APHA bezeichnet eine Farbzahl nach der American Public Health Assosiation nach Römpp, Chemielexikon, Georg Thieme Verlag Stuttgart, 1995.

FL%: bezeichnet den in "Flächen-%" angegebenen Anteil des jeweiligen Ethers bestimmt durch Gaschromatographie (GC). In diesem Fall wurde eine DB-Wax Säule mit 0,25 µm Innendurchmesser verwendet. Meßprogramm: Starttemperatur 50°C isotherm für 5 min., 50°C-240°C mit 10K/min., Temperatur des Detektors (FID) 250°C

Polymere Bestandteile (GPC): Die Berechnung polymerer Bestandteile erfolgte mit Gel-Permeationschromatographie.
Die Angabe polymere Bestandteile (GPC) = $\frac{FL_{Pr}}{C_{Pr}}$ bezeichnet die Menge polymerer Bestandteile gemessen in mV·s·ml/mg. $FL_{Pr.}$ bezeichnet die gefundene Fläche [mV·s] des an der Ausschlußgrenze eluierenden Peaks der Probe (hochmolekularer Anteil). $C_{Pr.}$ bezeichnet die Einwaage der Probe in 1 ml Eluent [mg/ml].
Der Wert wurde nach der aufgeführten Anzahl von Wochen nach dem Zeitpunkt der Zugabe des zu testenden Stabilisationsmittels zu dem Ether aufgenommmen. Die Lagerung erfolgte bei den Proben, die mit GPC analysiert wurden, bei 50°C soweit nichts anderes bestimmt ist.

T: Temperatur, die bei den Lagerungsversuchen vorlag. Für die Bestimmungen der diversen Meßparameter wurden nach entsprechenden Zeiten der Meßprobe entsprechende Aliquots entnommen und die Probe daraufhin mit Schutzgas überschichtet und abermals gelagert.

Tabelle 1.3 zeigt die Ergebnisse, die sich bei Lagerung von DVE-3 unter Zusatz von 0,01 % (w/w) Kaliumcarbonat bei einer Temperatur von 50°C bzw. 70°C ergeben.

Tabelle 1.3

| DVE-3 mit 0,01 % $K_2CO_3$ | 50°C | | | 70°C | | |
|---|---|---|---|---|---|---|
| | DVE-3-FL% | APHA | AA | DVE-3-FL% | APHA | AA |
| unmittelb. n. Zugabe | 98,9 | 2 | 39 | 98,9 | 3 | 40 |
| 2. Woche | 98,7 | 2 | 71 | 98,7 | 5 | 59 |
| 4. Woche | 98,78 | 7 | 110 | 98,8 | 26 | 110 |
| 6. Woche | 98,8 | 20 | 140 | 98,7 | 122 | 130 |
| 8. Woche | 98,0 | 35 | 76 | 99 | 219 | 79 |
| Polymere Bestandteile (GPC): 1256 (nach 8 Wochen) | | | | | | |

Tabelle 1.4 zeigt die Ergebnisse, die sich bei Lagerung von DVE-3 unter erfindungsgemäßem Zusatz von

0,01 % (w/w) Ammoniumcarbonat bei einer Temperatur von 50°C bzw. 70°C ergeben.

Tabelle 1.4

| DVE-3 mit 0,01 % $(NH_4)_2CO_3$ | 50°C | | | 70°C | | |
|---|---|---|---|---|---|---|
| | DVE-3-FL% | APHA | AA | DVE-3-FL% | APHA | AA |
| unmittelb. n. Zugabe | 98,8 | n.b. | n.b. | 98,8 | n.b. | n.b. |
| 2. Woche | 98,8 | 37 | 21 | 98,8 | 225 | 16 |
| 4. Woche | 98,98 | 16 | 37 | 98,93 | 300 | 29 |
| 6. Woche | 98,9 | 63 | 62 | 98,85 | 286 | 45 |
| 8. Woche | 98,6 | 71 | 91 | 98,7 | 360 | 46 |
| Polymere Bestandteile (GPC): 3650 (nach 4 Wochen) | | | | | | |

Tabelle 1.5 zeigt die Ergebnisse mit 0,01 % (w/w) Kaliumhydroxid

Tabelle 1.5

| DVE-3 mit 0,01 % KOH | 50°C | | | 70°C | | |
|---|---|---|---|---|---|---|
| | DVE-3-FL-% | APHA | AA | DVE-3-FL% | APHA | AA |
| unmittelb. n. Zugabe | 98,9 | 2 | 40 | 98,9 | 3 | 40 |
| 2. Woche | 98,7 | 265 | 24 | 98,7 | 186 | 46 |
| 4. Woche | 98,56 | 175 | 61 | 98,8 | 205 | 100 |
| 6. Woche | 98,9 | 166 | 91 | 98,9 | 418 | 140 |
| 8. Woche | 98,9 | 131 | 72 | 98,9 | 597 | 100 |
| Polymere Bestandteile (GPC): 6873 (nach 4 Wochen) | | | | | | |

Tabelle 1.6 zeigt die Ergebnisse mit 0,01 % (w/w) Natriumhydroxid

Tabelle 1.6

| DVE-3 mit 0,01% NaOH | 50°C | | | 70°C | | |
|---|---|---|---|---|---|---|
| | DVE-3-FL% | APHA | AA | DVE-3-FL% | APHA | AA |
| unmittelb. n. Zugabe | 99,58 | 5 | 18 | 99,58 | 5 | 18 |
| 2. Woche | 99,52 | 3 | 110 | 99,58 | 16 | 63 |
| 4. Woche | 99,33 | 13 | 170 | 99,29 | 69 | 94 |

Es ist ersichtlich, daß die erfindungsgemäße Stabilisatorsubstanz Ammoniumcarbonat die vorzeitige Polymerisation von Triethylenglykoldivinylether wirksam unterdrückt, wobei die Farbverschiebung wesentlich geringer ausfällt als bei dem Einsatz von Kaliumhydroxid. Zudem wird durch Ammoniumcarbonat die sich in der Bildung von Acetaldehyd äußernde Zersetzung von Triethylenglykoldivinylether wirksam verlangsamt.

**Beispiel 2**

In Beispiel 2 wird wie in Beispiel 1 verfahren, jedoch wird Triethylenglykoldivinylether durch 4-Hydroxybutylvinylether (HBVE) ersetzt. Angaben über Meßverfahren sind entsprechend übertragbar.

In Tabelle 2.1 werden die Ergebnisse bei Zusatz von 0,01 % (w/w) Kaliumcarbonat bei 50°C bzw. 70°C gezeigt.

Tabelle 2.1

| HBVE mit 0,01% $K_2CO_3$ | 50°C | | | 70°C | | |
|---|---|---|---|---|---|---|
| | HBVE-FL% | APHA | AA | HBVE -FL% | APHA | AA |
| unmittelb. n. Zugabe | 98,85 | n.b. | n.b. | 99,06 | n.b. | n.b. |
| 2. Woche | 98,62 | 11 | 130 | 98,52 | 18 | 270 |
| 4. Woche | 98,60 | 16 | 250 | 98,70 | 45 | 52 |
| 6. Woche | 98,87 | 13 | 340 | 98,00 | 16 | 580 |

Tabelle 2.1 (fortgesetzt)

| HBVE mit 0,01% $K_2CO_3$ | 50°C | | | 70°C | | |
|---|---|---|---|---|---|---|
| | HBVE-FL% | APHA | AA | HBVE -FL% | APHA | AA |
| 8. Woche | 98,70 | 11 | | 97,00 | 9 | |
| Polymere Bestandteile (GPC): 104 (nach 6 Wochen) | | | | | | |

In Tabelle 2.2 werden die Ergebnisse bei erfindungsgemäßem Zusatz von 0,01 % (w/w) Ammoniumcarbonat gezeigt.

Tabelle 2.2

| HBVE mit 0,01 % $(NH_4)_2CO_3$ | 50°C | | | 70°C | | |
|---|---|---|---|---|---|---|
| | HBVE -FL% | APHA | AA | HBVE-FL% | APHA | AA |
| unmittelb. n. Zugabe | 98,20 | n.b. | n.b. | 98,80 | n.b. | n.b. |
| 2. Woche | 98,00 | 14 | 22 | 97,70 | 22 | 23 |
| 4. Woche | 97,52 | 22 | 24 | 97,24 | 43 | 28 |
| 6. Woche | 98,06 | 28 | 34 | 96,30 | 46 | 35 |
| 8. Woche | 97,20 | 38 | 46 | 95,70 | 67 | 53 |
| Polymere Bestandteile (GPC): 52 (nach 6 Wochen) | | | | | | |

In Tabelle 2.3 werden die Ergebnisse bei Zusatz von 0,01 % (w/w) Ammoniumcarbamat gezeigt.

Tabelle 2.3

| HBVE mit 0,01 % A-carbamat | 50°C | | | 70°C | | |
|---|---|---|---|---|---|---|
| | HBVE-FL% | APHA | AA | HBVE-FL% | APHA | AA |
| unmittelb. n. Zugabe | 96,20 | n.b. | n.b. | 95,9 | n.b. | n.b. |
| 2. Woche | 96,10 | 17 | 41 | 94,9 | 55 | 39 |
| 4. Woche | 95,94 | 27 | 48 | 94,05 | 115 | 45 |
| 6. Woche | 96,40 | 35 | 61 | 92,5 | 141 | 71 |
| 8. Woche | 96,00 | 59 | 75 | 91,8 | 211 | 80 |
| Polymere Bestandteile (GPC): 51 (nach 6 Wochen) | | | | | | |

Es ist ersichtlich, daß die erfindungsgemäßen Stabilisatorsubstanzen Ammoniumcarbonat und Ammoniumcarbamat die vorzeitige Polymerisation von 4-Hydroxybutylvinylether wesentlich besser unterdrückt als die bekannte Stabilisatorsubstanz Kaliumcarbonat. Darüber hinaus zeigen die erfindungsgemäßen Stabilisatoren im Vergleich zu Kaliumcarbonat zwar eine etwas größere Farbverschiebung, jedoch unterdrücken sie die Bildung von Zersetzungsprodukten wie Acetaldehyd wesentlich effektiver als das bekannte Kaliumcarbonat. Kaliumhydroxid hat sich in diesem Versuch als völlig unbrauchbar erwiesen, so daß auf einen Vergleich verzichtet wurde.

**Beispiel 3**

In diesem Beispiel wurde Cyclohexylvinylether (CHVE) verwandt. Ansonsten wurde wie in den Beispielen 1 und 2 verfahren.

In Tabelle 3.1 sind die Ergebnisse für Cyclohexylvinylether ohne Stabilisator bei Raumtemperatur aufgelistet.

Tabelle 3.1

| CHVE unstabilisiert bei Raumtemperatur | | | |
|---|---|---|---|
| | CHVE-3-FL% | APHA | AA |
| unmittelb. n. Zugabe | 99,85 | <5 | 120 |
| 4. Woche | 99,79 | 5 | 310 |
| 8. Woche | 99,78 | 3 | 350 |
| 12. Woche | 99,78 | 8 | 590 |

Tabelle 3.1 (fortgesetzt)

| CHVE unstabilisiert bei Raumtemperatur | | | |
|---|---|---|---|
| | CHVE-3-FL% | APHA | AA |
| 16. Woche | 99,77 | <5 | 770 |
| 20. Woche | 99,74 | 3 | 640 |
| 24. Woche | 99,73 | <5 | n.b. |
| 28. Woche | 99,57 | 5 | 510 |
| Polymerisierbare Bestandteile (GPC): 881 (nach 16 Wochen, Raumtemperatur) | | | |

In Tabelle 3.2 werden die Ergebnisse bei Zusatz von 0,1 % (w/w) Kaliumcarbonat gezeigt.

Tabelle 3.2

| CHVE mit 0,1 % $K_2CO_3$ | 50°C | | |
|---|---|---|---|
| | CHVE-FL% | APHA | AA |
| unmittelb. n. Zugabe | 99,82 | 3 | 10 |
| 2. Woche | 99,72 | 5 | 180 |
| 4. Woche | 99,7 | 11 | 220 |
| 6. Woche | 99,64 | 12 | 250 |
| 8. Woche | 99,7 | 29 | 170 |
| 10. Woche | 99,7 | 33 | 200 |
| Polymerisierbare Bestandteile GPC: 173 (nach 6 Wochen) | | | |

In Tabelle 3.3 werden die Ergebnisse bei erfindungsgemäßem Zusatz von 0,01 % (w/w) Ammoniumcarbonat gezeigt.

Tabelle 3.3

| CHVE mit 0,01 % $(NH_4)_2CO_3$ | 50°C | | | 70°C | | |
|---|---|---|---|---|---|---|
| | CHVE-FL% | APHA | AA | CHVE-FL% | APHA | AA |
| unmittelb. n. Zugabe | 99,8 | <5 | 16 | 99,8 | <5 | 16 |
| 2. Woche | 99,7 | 3 | 12 | 99,8 | 8 | 17 |
| 4. Woche | 99,7 | 10 | 23 | 99,74 | 25 | 26 |
| 6. Woche | 99,66 | 14 | 19 | 99,7 | 33 | 20 |
| 8. Woche | 99,5 | 29 | 56 | 99,5 | | n.b. |
| Polymerisierbare Bestandteile (GPC): 60 (nach 6 Wochen) | | | | | | |

In Tabelle 3.4 werden die Ergebnisse mit 0,01 % (w/w) Ammoniumcarbamat gezeigt.

Tabelle 3.4

| CHVE mit 0,01 % A-carbamat | 50°C | | | 70°C | | |
|---|---|---|---|---|---|---|
| | CHVE-FL% | APHA | AA | CHVE-FL% | APHA | AA |
| unmittelb. n. Zugabe | 99,8 | <5 | 16 | 99,8 | <5 | 16 |
| 2. Woche | 99,74 | 6 | 20 | 99,76 | 19 | 10 |
| 4. Woche | 98,73 | 13 | 40 | 99,71 | 41 | 22 |
| 6. Woche | 98,73 | 13 | 45 | 99,55 | 91 | 20 |
| 8. Woche | 99,73 | 22 | 56 | 97,7 | n.b. | n.b. |
| Polymere Bestandteile (GPC): 125 (nach 6 Wochen) | | | | | | |

In Tabelle 3.5 werden die Ergebnisse bei Zusatz von 0,01 % (w/w) Kaliumhydroxid aufgeführt

Tabelle 3.5

| CHVE mit 0,1 % KOH | 50°C | | |
|---|---|---|---|
| | CHVE-FL% | APHA | AA |
| unmittelb. n. Zugabe | 99,82 | 3 | 10 |
| 2. Woche | 99,72 | 5 | 170 |
| 4. Woche | 99,7 | 11 | 190 |
| 6. Woche | 99,65 | 24 | 230 |
| 8. Woche | 99,7 | 46 | 130 |
| 10. Woche | 99,7 | 44 | 170 |
| Polymerisierbare Bestandteile (GPC): 151 (nach 6 Wochen) | | | |

Die erfindungsgemäßen Stabilisatorsubstanzen Ammoniumcarbonat und Ammoniumcarbamat unterdrücken die Polymerisation von Cyclohexylvinylether wesentlich effektiver als die Stabilisatorsubstanzen des Standes der Technik, Kaliumcarbonat und Kaliumhydroxid. Die erfindungsgemäßen Stabilisatorsubstanzen führen zu einer in etwa vergleichbaren Farbverschiebung wie die Stabilisatorsubstanzen Kaliumhydroxid und Kaliumcarbonat, jedoch hemmen sie die Zersetzung von Cyclohexylvinylether zu Acetaldehyd wesentlich effektiver.

**Patentansprüche**

**1.** Zusammensetzung von Vinylverbindungen enthaltend

a) mindestens eine polymerisierbare Verbindung, die mindestens eine O-Vinylgruppe oder eine N-Vinylgruppe aufweist, und
b) mindestens ein Ammoniumsalz der allgemeinen Formel (I)

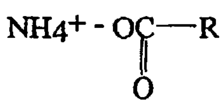

(I),

wobei R $NH_2$, $-O^- NH_4^+$, $C_1$-$C_{18}$-Alkyl, $C_6$-$C_{18}$-Cycloalkyl, oder $C_7$-$C_{18}$-Alkylcycloalkyl bedeutet.

**2.** Zusammensetzung nach Anspruch 1, **dadurch gekennzeichnet, daß** es sich bei dem Ammoniumsalz der Komponente b) um $(NH_4)_2CO_3$ oder um $NH_4NH_2CO_2$ handelt.

**3.** Zusammensetzung nach Anspruch 1 oder 2, **dadurch gekennzeichnet, daß** mindestens eine polymerisierbare Verbindung der Komponente a) unter die allgemeine Formel (II) fällt

$$CH_2{=}CH—X—R^1 \qquad \text{(II)},$$

wobei die Symbole die folgende Bedeutung haben

X O oder $NR^2$,

$R^1$

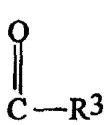

oder $R^3$,

$R^2$ Wasserstoff oder $C_1$-$C_4$-Alkyl oder gemeinsam mit $R^3$ eine $C_3$-, $C_4$- oder$C_5$-Alkylenbrücke oder Alkenylenbrücke bildend, in welchen bis zu zwei nicht-benachbarte $CH_2$-Gruppen durch NH, N($C_1$-$C_4$-Alkyl), N ($C_6$-$C_{10}$-Aryl) oder Sauerstoff und bis zu zwei nicht-benachbarte CH-Gruppen durch N ersetzt sein können und

$R^3$ Wasserstoff oder eine einfach oder mehrfach Hydroxy-substituierte oder Vinyloxy-substituierte oder unsubstituierte $C_1$-$C_{16}$-Alkyl-, $C_6$-$C_{16}$-Cycloalkyl- oder $C_1$-$C_4$-Alkyl-$C_6$-$C_{12}$-cycloalkylgruppe oder zusammen mit $R^2$ eine $C_3$-, $C_4$- oder $C_5$-Alkylenbrücke oder Alkenylenbrücke darstellend, in welchen bis zu zwei nicht-benachbarte $CH_2$-Gruppen durch NH, N($C_1$-$C_4$-Alkyl), N($C_6$-$C_{10}$-Aryl) oder Sauerstoff und bis zu zwei nicht-benachbarte CH-Gruppen durch N ersetzt sein können.

**4.** Zusammensetzung nach Anspruch 3, **dadurch gekennzeichnet, daß** X Sauerstoff bedeutet.

**5.** Zusammensetzung nach Anspruch 4, **dadurch gekennzeichnet, daß** die polymerisierbaren Verbindung der Komponente a) Triethylenglykoldivinylether (DVE), 4-Hydroxybutylvinylether (HBVE) oder Cyclohexylvinylether oder ein Gemisch daraus ist.

**6.** Zusammensetzung nach Anspruch 3, **dadurch gekennzeichnet, daß** X-$R^1$ $NH_2COCH_3$, N-Imidazolyl, N-Pyrrolidinonyl oder N-Caprolactamyl bedeutet.

**7.** Verwendung eines Ammoniumsalzes der allgemeinen Formel (I) gemäß Anspruch 1 als Zusatz zu polymerisierbaren Verbindungen, die mindestens eine O-Vinylgruppe oder eine N-Vinylgruppe aufweisen.

**8.** Verwendung von $(NH_4)_2CO_3$ oder um $NH_4NH_2CO_2$ als Zusatz zu polymerisierbaren Verbindungen, die mindestens eine O-Vinylgruppe oder eine N-Vinylgruppe aufweisen.

**9.** Verfahren zur Verhinderung der vorzeitigen Polymerisation von Zusammensetzungen, die polymerisierbare Verbindungen enthalten, die mindestens eine O-Vinylgruppe oder eine N-Vinylgruppe aufweisen, **dadurch gekennzeichnet, daß** den Zusammensetzungen ein Ammoniumsalz der allgemeinen Formel (I) gemäß Anspruch 1 zugesetzt wird.

**10.** Verfahren nach Anspruch 9, bei dem als Ammoniumsalz $(NH_4)_2CO_3$ oder $NH_4NH_2CO_2$ eingesetzt wird.

## Claims

**1.** A composition of vinyl compounds, comprising

a) at least one polymerizable compound which has at least one O-vinyl group or one N-vinyl group, and
b) at least one ammonium salt of the formula (I)

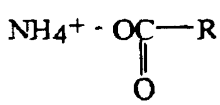

(I),

where R is $NH_2$, $-O^- NH_4^+$, $C_1$-$C_{18}$-alkyl, $C_6$-$C_{18}$-cycloalkyl, or $C_7$-$C_{18}$-alkylcycloalkyl.

**2.** A composition as claimed in claim 1, wherein the ammonium salt of component b) is $(NH_4)_2CO_3$ or $NH_4NH_2CO_2$.

**3.** A composition as claimed in claim 1 or 2, wherein at least one polymerizable compound of component a) is covered by the formula (II)

$$CH_2=CH—X—R^1 \quad (II),$$

where

X is O or $NR^2$,

$R^1$ is

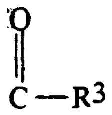

or $R^3$,

$R^2$ is hydrogen or $C_1$-$C_4$-alkyl or, together with $R^3$, is a $C_3$, $C_4$, or $C_5$ alkylene bridge or alkenylene bridge, in which up to two non-adjacent $CH_2$ groups may have been replaced by NH, N($C_1$-$C_4$-alkyl), N($C_6$-$C_{10}$-aryl) or oxygen, and up to two non-adjacent CH groups may have been replaced by N, and

$R^3$ is hydrogen or a singly or multiply hydroxyl-substituted or vinyloxy-substituted or unsubstituted $C_1$-$C_{16}$-alkyl-, $C_6$-$C_{16}$-cycloalkyl- or $C_1$-$C_4$-alkyl-$C_6$-$C_{12}$-cycloalkyl group or, together with $R^2$, is a $C_3$, $C_4$ or $C_5$ alkylene bridge or alkenylene bridge, in which up to two non-adjacent $CH_2$ groups may have been replaced by NH, N($C_1$-$C_4$-alkyl), N($C_6$-$C_{10}$-aryl) or oxygen, and up to two non-adjacent CH groups may have been replaced by N.

**4.** A composition as claimed in claim 3, wherein X is oxygen.

**5.** A composition as claimed in claim 4, wherein the polymerizable compound of component a) is triethylene glycol divinyl ether (DVE), 4-hydroxybutyl vinyl ether (HBVE) or cyclohexyl vinyl ether or a mixture of these.

**6.** A composition as claimed in claim 3, wherein X-$R^1$ is $NH_2COCH_3$, N-imidazolyl, N-pyrrolidinonyl or N-caprolactamyl.

**7.** The use of an ammonium salt of the formula (I) as claimed in claim 1 as additive to polymerizable compounds which have at least one O-vinyl group or one N-vinyl group.

**8.** The use of $(NH_4)_2CO_3$ or $NH_4NH_2CO_2$ as additive to polymerizable compounds which have at least one O-vinyl group or one N-vinyl group.

**9.** A process for inhibiting premature polymerization of compositions which comprise polymerizable compounds which have at least one O-vinyl group or one N-vinyl group, which comprises adding to the compositions an ammonium salt of the formula (I) as claimed in claim 1.

**10.** A process as claimed in claim 9, in which the ammonium salt used is $(NH_4)_2CO_3$ or $NH_4NH_2CO_2$.

## Revendications

**1.** Composition de dérivés vinyliques contenant :

(a) Au moins un composé polymérisable contenant au moins un groupe 0-vinyle ou un groupe N-vinyle et
(b) Au moins un sel d'ammonium de formule générale I

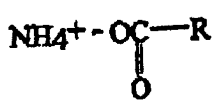

(I),

dans laquelle R représente $NH_2$, $-0^- NH_4^+$, un groupe alkyle en C1-C18, cycloalkyle en C6-C18 ou alkylcycloalkyle en C7-C18.

**2.** Composition selon la revendication 1, **caractérisée en ce que** le sel d'ammonium du composant b) est $(NH_4)_2CO_3$ ou $NH_4NH_2CO_2$..

**3.** Composition selon la revendication 1 ou 2, **caractérisée en ce qu'**au moins un composé polymérisable du composant a) répond à la formule générale II

$$CH_2{=}CH—X—R^1 \qquad (II),$$

dans laquelle les symboles ont les significations suivantes :

X: O ou $NR^2$,

$R^1$

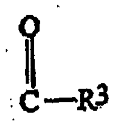

ou $R^3$,

$R^2$ l'hydrogène ou un groupe alkyle en C1-C4 ou bien $R^2$ et $R^3$ représentent ensemble un pont alylène ou alcénylène en C3, C4 ou C5 dans lequel jusqu'à deux groupes $CH_2$ non voisins peuvent être remplacés par NH, N(alkyle en C1-C4), N(aryle en C6-C10) ou l'oxygène et jusqu'à deux groupes CH non voisins peuvent être remplacés par N, et

$R^3$ l'hydrogène ou un groupe alkyle en C1-C16, cycloalkyle en C6-C16 ou (alkyle en C1-C4)cycloalkyle en C6-C12 non substitué ou portant un ou plusieurs substituants hydroxy ou vinyloxy, ou bien $R^3$ et $R^2$ forment ensemble un pont alkylène ou alcénylène en C3, C4 ou C5 dans lequel jusqu'à deux groupes $CH_2$ non voisins peuvent être remplacés par NH, N(alkyle en C1-C4), N-(aryle en C6-C10) ou l'oxygène et jusqu'à deux groupes CH non voisins peuvent être remplacés par N.

**4.** Composition selon la revendication 3, **caractérisée en ce que** X représente l'oxygène.

**5.** Composition selon la revendication 4, **caractérisée en ce que** le composé polymérisable du composant a) est l'éther divinylique du triéthylèneglycol (DVE), l'éther 4-hydroxybutylvinylique (HBVE) ou l'éther cyclohexylvinylique ou un mélange de ces composés.

**6.** Composition selon la revendication 3, **caractérisée en ce que** X-$R^1$ représente $NH_2COCH_3$, un groupe N-imidazolyle, N-pyrrolidinonyle ou N-caprolactamyle.

**7.** Utilisation d'un sel d'ammonium de formule générale I selon la revendication 1 en tant qu'additif à des composés polymérisables contenant au moins un groupe O-vinyle ou un groupe N-vinyle.

**8.** Utilisation de $(NH_4)_2CO_3$ ou de $NH_4NH_2CO_2$ en tant qu'additif à des composés polymérisables contenant au moins un groupe O-vinyle ou un groupe N-vinyle.

**9.** Procédé pour empêcher la polymérisation prématurée de compositions contenant des composés polymérisables à au moins un groupe O-vinyle ou un groupe N-vinyle, **caractérisé en ce que** l'on ajoute à ces compositions un sel d'ammonium de formule générale I selon la revendication 1.

**10.** Procédé selon la revendication 9, selon lequel on utilise en tant que sel d'ammonium $(NH_4)_2CO_3$ ou $NH_4NH_2CO_2$.